# EUROPEAN PATENT SPECIFICATION

(11) **EP 1 200 149 B1**
(45) Date of publication and mention of the grant of the patent: **20.09.2006**
(21) Application number: 00960184.0
(22) Date of filing: 21.07.2000
(51) Int. Cl.: A61M 15/00, A61J 3/07

(54) **DRY POWDER INHALER**
TROCKENPULVERINHALATOR
INHALATEUR A POUDRE SECHE

(30) Priority: 23.07.1999 US 145464 P; 22.05.2000 US 206123 P
(43) Date of publication of application: 02.05.2002
(60) Divisional application: 05077995.8
(73) Proprietor: MannKind Corporation, Valencia CA 91355 (US)
(72) Inventor: STEINER, Solomon, S., Mount Kisco, NY 10507 (US); POOLE, Trent, South Amherst, MA 01002 (US); FELDSTEIN, Robert, Yonkers, NY 10701 (US); FOG, Per, B., Bedford Hills, NY 10507 (US)
(74) Representative: Powell, Timothy John
(86) International application number: PCT/US2000/040454
(87) International publication number: WO 2001/007107

(56) References cited:
- EP-A- 0 308 637
- EP-A- 0 581 473
- EP-A- 0 666 085
- WO-A-96/22802
- WO-A-98/26827
- WO-A-98/41255
- WO-A1-95/05208
- DE-A- 3 639 836
- DE-A- 19 519 840
- GB-A- 2 072 536
- GB-A- 2 148 841
- US-A- 3 823 816
- US-A- 4 040 536
- US-A- 4 047 525
- US-A- 4 487 327
- US-A- 5 170 801
- US-A- 5 524 613
- US-A- 5 758 638
- US-A- 5 797 391
- US-A- 5 896 855

## Description

### FIELD OF THE INVENTION

The present invention is in the field of inhalers.

### BACKGROUND OF THE INVENTION

In the early 1970's it was found that certain medicines could be administered in dry-powder form directly to the lungs by inhalation through the mouth or inspiration through the nose. This process allows the medicine to bypass the digestive system, and may, in certain cases, allow smaller doses to be used to achieve the same results as orally ingested or injected medicines. In some cases, it provides a delivery technique that reduces side effects for medicines taken by other methods.

Inhaler devices typically deliver their medicinal in a liquid mist or a powder mist. The liquid mist is typically created by a chlorofluorocarbon propellant. However, with the ban on chlorofluorocarbons by the Montreal protocol, interest has turned to dry powder inhalers.

For a dry powder inhaler to work effectively, it must deliver fine particles of medicinal powder that do not agglomerate, and do not end up striking, and being absorbed by the patient's mouth or upper oropharyngeal region. Air flow must therefore not be too fast. Furthermore, it should not be difficult for a patient to load with medicine or to use with the proper technique. Current dry particle inhalers fail in one or more of these important criteria.

GB 2 072 536 discloses a dry powder inhaler comprising an intake section that is mechanically connected to a mouthpiece, wherein the intake section and mouthpiece each have a longitudinal axis, and wherein air flows through a passage extending from the intake section through the mixing section through the mouthpiece. Also the mechanical connection between the mouthpiece and themixing section comprises a swivel joint which allows the longitudinal axis of the intake section to be parallel to the longitudinal axis of the mouthpiece.

WO95/05208 discloses an inhalation device according to the preamble of claim 1, including a breath flow regulation arrangement. This arrangement shows a reduction in the flow rate of air delivered as a pressure differential caused by inhalation increases. The air flow regulation portion of the device may include a piston and spring combination.

### SUMMARY OF THE INVENTION

Described is a dry powder inhaler according to claim 1 comprising an intake section; a mixing section, and a mouthpiece. The mouthpiece may be connected by a swivel joint to the mixing section, and may swivel back onto the intake section and be enclosed by a cover. The intake chamber comprises a special piston with a tapered piston rod and spring, and one or more bleed-through orifices to modulate the flow of air through the device. The intake chamber further optionally comprises a feedback module to generate a tone indicating to the user when the proper rate of airflow has been achieved. The mixing section holds a capsule with holes containing a dry powder medicament, and the cover only can open when the mouthpiece is at a certain angle to the intake section. The mixing section further opens and closes the capsule when the intake section is at a certain angle to the mouthpiece. The mixing section is a Venturi chamber configured by protrusions or spirals to impart a cyclonic flow to air passing through the mixing chamber. The mouthpiece includes a tongue depressor, and a protrusion to contact the lips of the user to tell the user that the DPI is in the correct position. An optional storage section, with a cover, holds additional capsules. The cover for the mouthpiece, and the cover for the storage section may both be transparent magnifying lenses.

The capsules may be two-part capsules where each portion has apertures which correspond to apertures in the other half when each half is partially fitted to the other half, and fully fitted to the other half. All the apertures may be closed when the two halves are rotated around their longitudinal axes with respect to each other. Each capsule may have a unique key on each half that only fits with a particular inhaler.

Therefore it is an object of the invention to provide a dry particle inhaler that can fold into a compact form.

Therefore it is an object of the invention to provide a dry particle inhaler that can be loaded with medicament easily.

Therefore it is an object of the invention to provide a dry particle inhaler where the small writing on a capsule of medicament can be easily read.

Therefore it is an object of the invention to provide a dry particle inhaler where a capsule containing medicament can only be inserted when a person unfolds the inhaler for use.

Therefore it is an object of the invention to provide a dry particle inhaler where the air flow through the device is regulated.

Therefore it is an object of the invention to provide a dry particle inhaler to provide a means for indicating to the user when the air flow is at the correct rate.

Therefore it is an object of the invention to provide a dry particle inhaler where particles of drug are dispersed finely.

These and other objects of the invention will be readily apparent upon a reading of the present specification, claims and drawings.

### BRIEF DESCRIPTION OF THE SEVERAL VIEWS OF THE DRAWINGS

Figure 1 is a schematic view of the dry particle inhaler described herein.
Figure 2 is schematic view of the mouthpiece cover.
Figure 3 is schematic view showing the angle between the intake section and the mouthpiece.
Figure 4 is a schematic view of the dry particle inhaler, showing the storage section.
Figure 5 is a schematic view of the intake section of the dry particle inhaler, showing the flow regulator and the feedback module.
Figure 6 is a schematic view of the mixing section.
Figure 7 is a schematic view of a capsule to hold medicament.
Figure 8 is a schematic view of the mouthpiece.
Figure 9 is a perspective view of a specific embodiment of the dry particle inhaler in the closed position, with a capsule inserted into the mixing section, and extra capsules stored in the storage section.
Figure 10 is a perspective view of a specific embodiment of the dry particle inhaler showing a capsule being loaded in to the mixing section.
Figure 11 is a perspective view of a specific embodiment of the dry particle inhaler showing a capsule inserted into the mixing section, and the mouthpiece extended for use.
Figures 12, 13, 14, and 15 follow each other in temporal sequence.

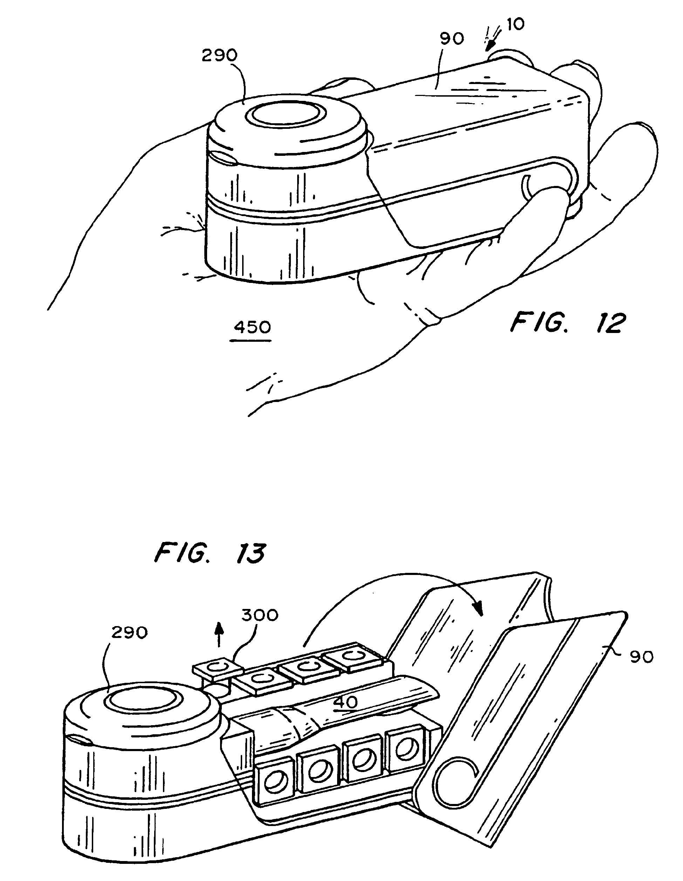

Figure 12 is a perspective view of a specific embodiment of the dry particle inhaler showing a closed mouthpiece cover.
Figure 13 is a perspective view of a specific embodiment of the dry particle inhaler showing an open mouthpiece cover.

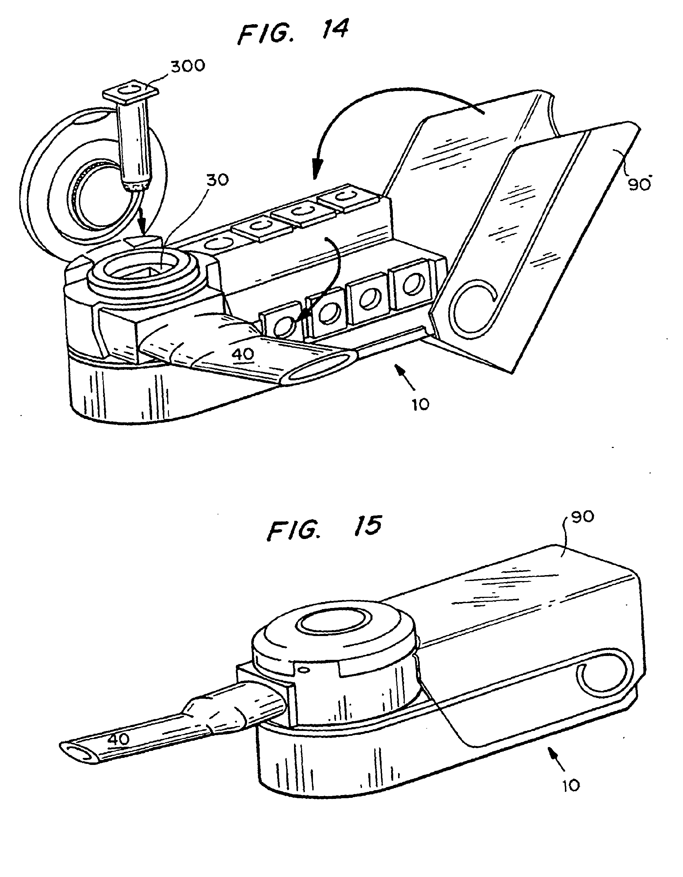

Figure 14 is a perspective view of a specific embodiment of the dry particle inhaler showing an open mouthpiece cover, an open mixing section cover, and a capsule about to be inserted into the mixing section.
Figure 15 is a perspective view of a specific embodiment of the dry particle inhaler showing the mouthpiece extended for use.

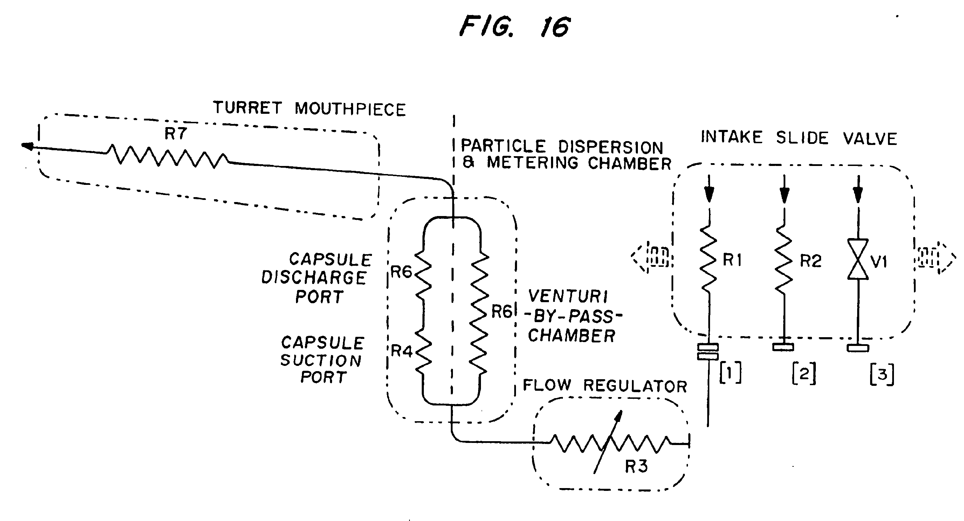

Figure 16 is a view of a pneumatic circuit, where air flows (fluid flows) are represented by their electrical equivalents.

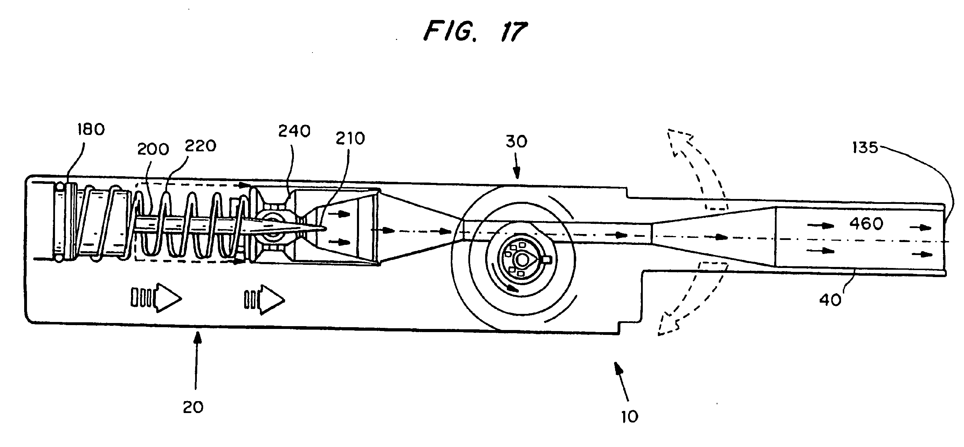

Figure 17 is a schematic view of the dry particle inhaler.

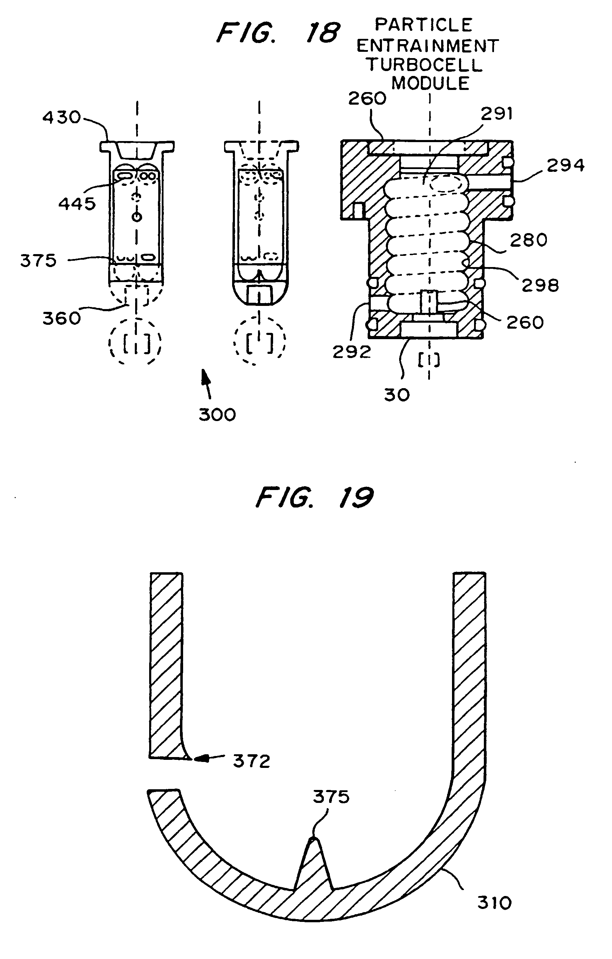

Figure 18 is a cutaway view of a capsule and a portion of the mixing section.
Figure 19 is a cutaway view of half of a capsule, showing a cone in the interior and a secondary hole with a chamfered, or beveled, edge.

### TABLE OF REFERENCE NUMBERS

- 10: dry powder inhaler device
- 20: intake section
- 30: mixing section
- 40: mouthpiece
- 50: air passage through dry powder inhaler device
- 60: longitudinal axis of intake section
- 70: longitudinal axis of mouthpiece section
- 80: swivel joint connecting mouthpiece and mixing section
- 90: cover for mouthpiece
- 100: protrusions on mouthpiece cover
- 110: depressions on dry particle inhaler cover to mate with protrusions on mouthpiece cover
- 120: tongue depressor on mouthpiece
- 130: protrusion on surface of mouthpiece to contact lips of device user
- 135: opening of mouthpiece to be fitted into user's mouth
- 140: intake port
- 150: flow regulator
- 160: bleed orifice
- 170: piston
- 180: piston head
- 190: piston rod
- 200: proximal portion of piston rod
- 210: distal portion of piston rod
- 220: spring
- 230: inner walls of intake section inner chamber
- 240: feedback module
- 250: mechanical fasteners in storage section
- 260: holder in mixing section for capsule
- 270: Venturi chamber
- 280: spiral shape or protrusions to impart cyclonic flow to air
- 290: cover for mixing chamber
- 291: interior of mixing section
- 292: air flow entrance to mixing section
- 294: air flow exit from mixing section
- 296: latch mechanism for mixing section cover
- 298: interior wall of mixing section
- 300: capsule
- 310: first tube
- 320: open end of first tube
- 330: closed end of first tube
- 340: long axis of first tube
- 350: protrusion on first tube
- 360: keying surface on first tube
- 370: secondary holes in first tube
- 372: chamfered edge of secondary hole
- 375: cone in interior of first tube
- 380: second tube
- 390: open end of second tube
- 400: closed end of second tube

- 410: long axis of second tube
- 420: protrusion on second tube
- 430: keying surface on second tube
- 440: secondary holes in second tube
- 445: cone in interior of second tube
- 450: hand of user
- 460: air flow direction
- 470: storage section
- 480: storage section cover

### DETAILED DESCRIPTION OF THE INVENTION

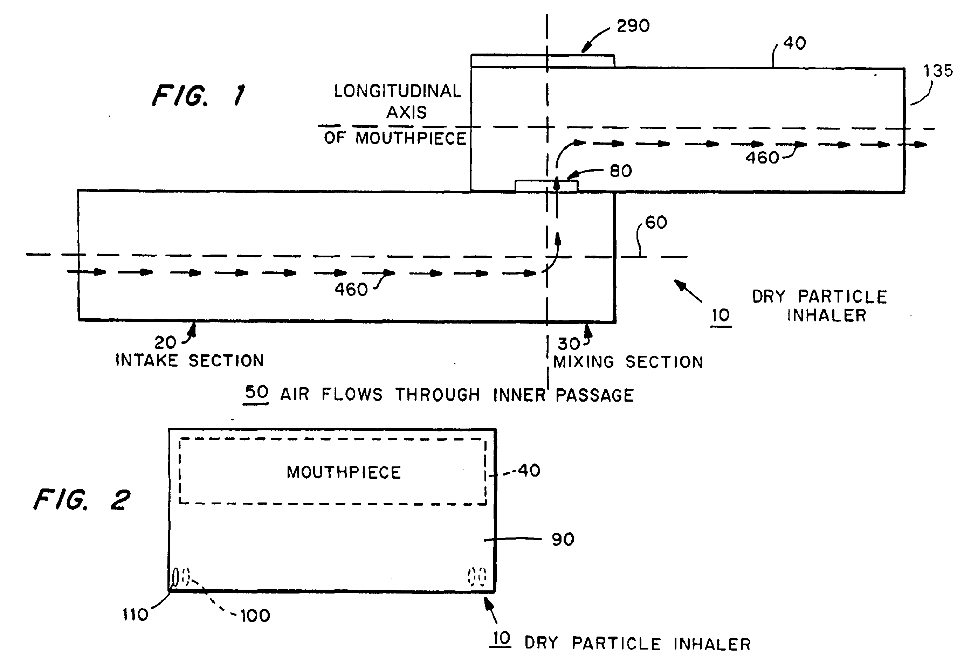

Figure 1 is a schematic drawing of the dry powder inhaler (10) described herein. It comprises an intake section (20), a mixing section (30) and a mouthpiece (40). An air passage (50) goes through the intake section (20), a mixing section (30) and a mouthpiece (40). A swivel joint (80) connects the mouthpiece (40) to the mixing section (30). The mixing section (20) has a cover (290) which may be a transparent magnifying lens. Arrow (460) shows the direction of air flow through the air passage (50) through the dry powder inhaler (10).

Figure 2 shows the mouthpiece cover (90) in the closed position over the dry particle inhaler (10). Protrusions (100) on the mouthpiece cover (90) mate with grooves or depressions (110) on the dry particle inhaler (10), to join the mouthpiece cover (90) to the dry particle inhaler (10).

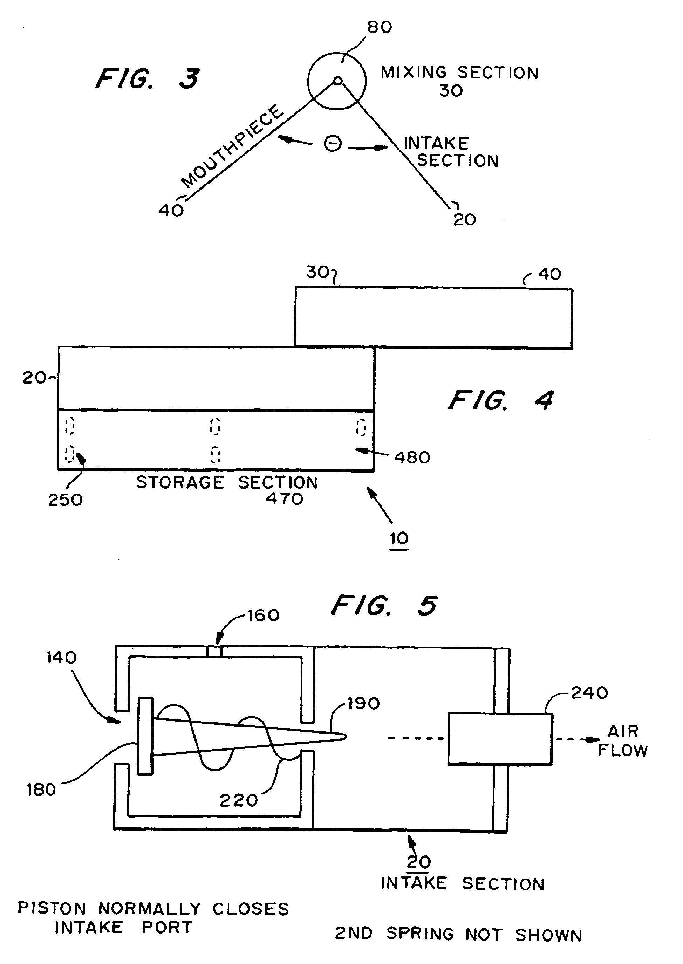

Figure 3 is a schematic of the showing the mouthpiece (40) and the intake section (20) as represented by the longitudinal axis of the mouthpiece (70) and the longitudinal axis of the intake section (60). The swivel joint (80) connecting the mouthpiece (40) to the intake section (20) at the mixing section (30) may be regarded as the vertex of the angle. The importance of the angle (here called theta) between these two longitudinal axes will be further explained.

Figure 4 shows the dry particle inhaler (10) with a storage section (470). Indicated as being inside the storage section (470) are mechanical fasteners (250) which operate to hold medicament capsules (300) (not shown in this Figure) in the storage section. In this embodiment, the storage section (470) is shown as appended to the intake section (20). The storage section has a cover (480) which may be a transparent magnifying lens, to allow the user to easily read writing on medicament capsules stored therein. The storage section cover (480) may swivel outward, or slide open on a track (not shown), or open by a variety of mechanisms known to those of skill in the art.

Figure 5 shows the intake section (20) of the dry particle inhaler (10). The direction of air flow is shown by the arrow (460). Air is admitted through an intake port (140) and one or more bleed orifices (160) [The bleed orifices may also be styled as secondary ambient air intake ports]. The piston (170) normally covers the intake port (140). When the user (not shown) inspires, the piston head (180) is drawn backwards, at a steady rate modulated by the spring (220). The spring (220) is fixed to the piston (170) and the inner wall (230) of the intake section chamber. Thus the rate of air flow is controlled. The air flow is further controlled by the tapering of the piston rod (190), past which the air flows. For further control of the air flow, a second spring (not shown) may also control the rate of movement of the piston (170).

The piston (170) and spring (220) combination allow the user (not shown) to generate a vacuum in his lungs before the intake port (140) opens. Thus, by the time enough vacuum is generated to open the intake port (140), there will be sufficient air flow at a sufficient rate in the dry particle inhaler (10) to draw most of the medicament in the capsule (not shown) out of the inhaler into the proper place in the lungs of the user.

A feedback module (240) generates a signal to the user (not shown), which tells the user whether he is inspiring at the correct rate. The signal may be an audible one, in one embodiment a tone that is at a steady pitch when air flow is at a certain steady rate. In one embodiment of the dry particle inhaler (10), the signal is generated mechanically, such as be a musical reed. In another embodiment of the invention, the signal might be generated electronically, after electronic measurement of the air flow rate. The feedback module (240) would include a means for increasing or lessening the signal strength, or turning the signal off entirely. If the signal were generated by a reed, the mechanism for turning off the signal might be covering a bleed orifice which might admit the air flow generating the signal. If the signal were generated electronically, a simple push button or dial might turn on and off the signal.

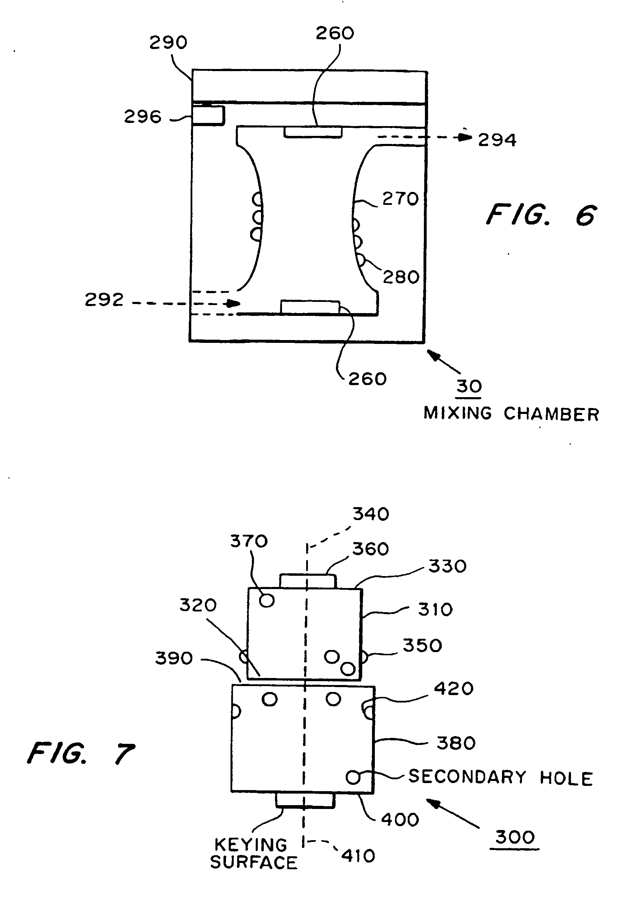

Figure 6 shows a schematic of the mixing section (30) of the present invention. The mixing section has a cover (290), and a holder (260) for a medicament capsule (not shown). The holder (260) is a mechanism which grips and turns the capsule (not shown) to open and close it as the longitudinal axis (70) of the mouthpiece is rotated about the swivel joint (80) relative to the longitudinal axis (60)of the intake section. Such a mechanism may be straightforward: in a simplest embodiment, both the top and bottom halves (not shown) of the capsule could be fixed to their respective holders (260).

The Venturi chamber (270) speeds the flow of air near the capsule (not shown). Air flows in at (292), and out through (294). In one embodiment, air flows both through and around a capsule (not shown) holding a dry powder medicament. The special shape of the Venturi chamber (270), which further includes protrusions or spiral shapes (280), imparts a cyclonic flow to the air passing through the mixing section (30). This helps to de-agglomerate particles of dry powder. The spiral shape of the interior of the mixing section (291) can be two separate spirals, in one embodiment of the invention. Mixing section (30) therefore provides the means whereby air flow is speeded up to suspend dry particles in air and de-agglomerate them, and then slow the air flow somewhat while the particles are still suspended in air. The cover (290) for the mixing section (30) may be a transparent magnifying lens, so that any writing on the capsule (not shown) may be read easily.

In one embodiment of the dry particle inhaler (10), the cover (290) of the mixing section may not be opened unless the longitudinal axis (70) of the mouthpiece forms a certain angle with the longitudinal axis (60) of the intake section, with the vertex of the angle being the swivel joint (80) connecting the mouthpiece (40) and the mixing section (30). The latch mechanism (296) for the cover (290) of the mixing section can accomplish this, by any of several mechanical means known to those of ordinary skill in the art. In the simplest embodiment, a catchment (not shown) in the cover (290) for the mixing chamber would be engaged by a slip ring (not shown) on the mixing section which was only a certain number of degrees of a circle. When the mouthpiece (40) were rotated enough relative to the intake section (20), the slip ring (not shown) would no longer engage the catchment (not shown). In one embodiment, the user could open the cover (290) when the angle were between approximately ninety and one-hundred and eighty degrees.

Figure 7 shows a medicament capsule (300) for use with an inhaler, be it a dry powder inhaler (10), or a liquid mist inhaler. The capsule (300) has two halves which fit together, here styled a first tube (310) and a second tube (380). Each tube has an open end (320, 390), and a closed end (330, 400). Each tube also has a long axis (340, 410). In addition, each.tube has a number of secondary holes (370, 440). The first tube (310) fits inside the second tube (380) snugly. A protrusion (350) on the outer surface of the first tube (310) can slide past a corresponding protrusion (420) on the inner surface of the second tube (380). This locks the first tube (310) to the second tube (380). Therefore the first tube (310) and the second tube (380) have both an unlocked and a locked position. In the unlocked position, at least one secondary hole (370) in the first tube aligns with at least one secondary hole (440) in the second tube. This permits introduction of a medicament (not shown) into the capsule through the aligned secondary holes (370, 440). The first tube (310) may then be locked to the second tube (380). When a user (not shown) is ready to use a capsule (300), he simply places it in the holder (260) in the mixing section (30), and closes the cover (290). When the holder (260) rotates the first tube (310) around its long axis (340) relative to the second tube (380) and its long axis (410) (the axes are now coincident), that causes at least two secondary holes (370) in the first tube to align with at least two secondary holes (440) in the second tube. Air can now pass in, through, and out of the capsule (300), releasing the medicament contained therein. In one embodiment of the inhaler, the capsule (300) might open when the angle between the longitudinal axis (70) of the mouthpiece section, the vertex of the swivel joint (80), and the longitudinal axis (70) of the mouthpiece section were between one hundred and seventy and one-hundred and eighty degrees. This rotation of the mouthpiece (40) relative to the intake section (20) would cause a corresponding rotation of the first tube (310) about its long axis (340) relative to the second tube (380) and its long axis (410).

In one embodiment of the invention, several protrusions on the surfaces of the first tube or the second tube might provide a variety of locking positions. Similarly, a variety of secondary holes in the first and second tubes might provide a variety of rotational positions aligning or not aligning secondary holes on the first and second tubes.

The capsules described herein permit the introduction of liquid or gel medicament which can be dried in the capsule, creating a powder. This permits the accurate production of very small amounts of powdered medicament in a capsule, since it can be formed from a larger volume of accurately metered liquid or gel medicament. This permits very accurate microdosing. In addition, chemical reactions and drug mixtures may be made directly in the capsules described herein, then the resulting formulation dried.

In one embodiment of the capsule (300), one or more of the secondary holes (370, 440) used to admit air to the capsule is oval-shaped (elliptical). In one embodiment of the invention, the ratio of the long axis of the ellipse to the shorter axis may be between 1:1 and 3:1, and may be 2:1. This ratio may be called a vertical aspect ratio. In one embodiment of the invention, the intersection of the surface defining one or more of the secondary holes (370, 440) and the surface defining the interior of the capsule (300) meet in a chamfered, or beveled, edge. This chamfered edge creates a vortex when air flows through the secondary holes (370, 440).

Each capsule (300) also has a keying surface (or fastening mechanism) on the closed end (330) of the first tube and the closed end (400) of the second tube comprising the capsule. The keying surface (360) on the first tube may be different from the keying surface (430) on the second tube. That permits easy tactile and visual identification of the orientation of the capsule. It also permits a system where each drug formulation in a capsule (300) corresponds to a dry particle inhaler (10), so users cannot mix up drugs. In one embodiment of the invention, the keying surface (360) of the first tube mates with a keying surface (430) of a different second tube, or the mechanical fasteners (250) of the storage section (470). This permits easy storage of the capsules (300) in the storage section (470).

Figure 18 shows a medicament capsule (300), with a keying surface (360) on the first tube and a keying surface (430) on the second tube. It also shows a cutaway view of the mixing section (30) and the air flow entrance (292) to the mixing section and the air flow exit (294) to the mixing section. A spiral shape (280) is given to the interior walls (298) of the mixing section, to impart a cyclonic flow to air passing through. The air flow entrance (292) and air flow exit (294) in this embodiment are tangential to the imaginary tube we might call the mixing section interior (291). That is to say, if a radius were drawn perpendicular to the long axis of the tube, and a tangent line were drawn to the circle perpendicular to the radius, the air flow would exit the mixing section along that tangent line. The tangential air flow exit (294) increases the velocity of the air flow, and thus helps disperse the medicament particles. As can be seen from Figure 18, the mixing section interior (291) is sized to accommodate a medicament capsule (300). Keying mechanisms (360, 430) are shaped to mate with holder (260) in the mixing section. Capsules according to the present invention may have a number of shapes, including ovoid and rectangular shapes. A variety of shapes of protrusions and slots may also be employed as keying surfaces. For instance, a keying surface might be a rectangular block, and a capsule holder might have a rectangular orifice. Alternatively, a keying surface might be triangular, hexagonal, Z-shaped, C-shaped, etc., and the holder would have the correspondingly shaped aperture:

Figure 18 also shows one embodiment of the capsule (300) where a cone (375) is located in the interior of the first tube, and a cone (445) is located in the interior of the second tube. These cones (375, 445) cause the air flow within the capsule to be cyclonic, aiding in mixing the medicament particles with the air. A cone is shown herein, but other cyclone-creating structures are contemplated by the present invention.

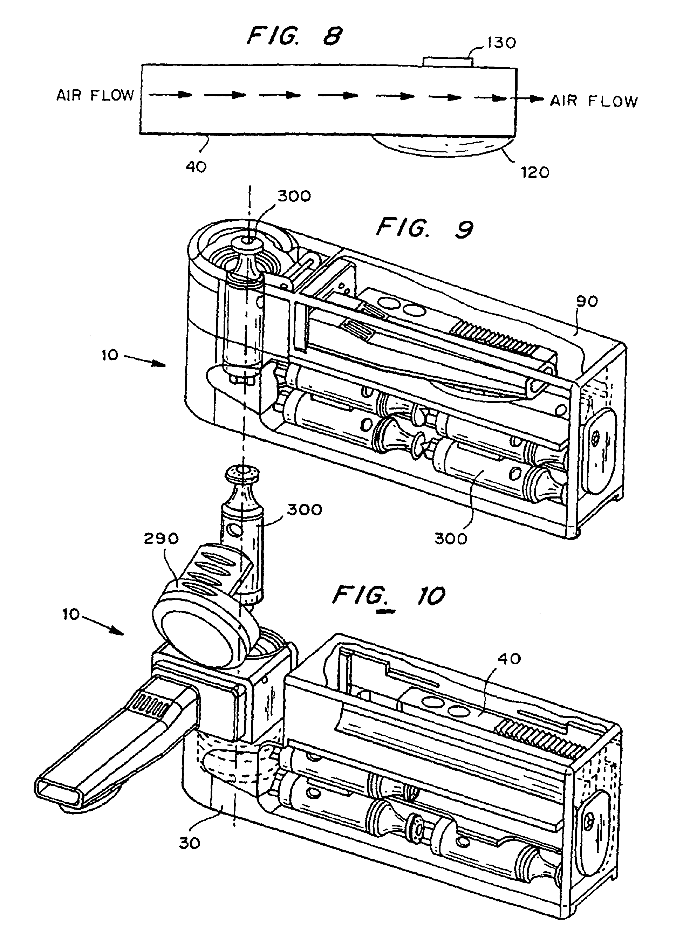

Figure 8 shows the mouthpiece (40) of the dry particle inhaler (10). It has a protrusion (130) on its surface to contact the lips of a user (not shown). This helps the user place the mouthpiece correctly in his mouth. The mouthpiece (40) also includes a tongue depressor (120), which may have a bulbous shape. The mouthpiece (40) is long enough that it fits approximately midway into the user's mouth (not shown). This permits greater delivery of medicament to the lungs, and less delivery to the oral cavity. The mouthpiece (40) has a particular aspect ratio of its inner channel (50) (see Figure 17). This slows the air passing through the channel so that the air borne particulates do not end up striking the back of the user's throat. However, the air is not slowed so much that the particulates settle out of the air flow.

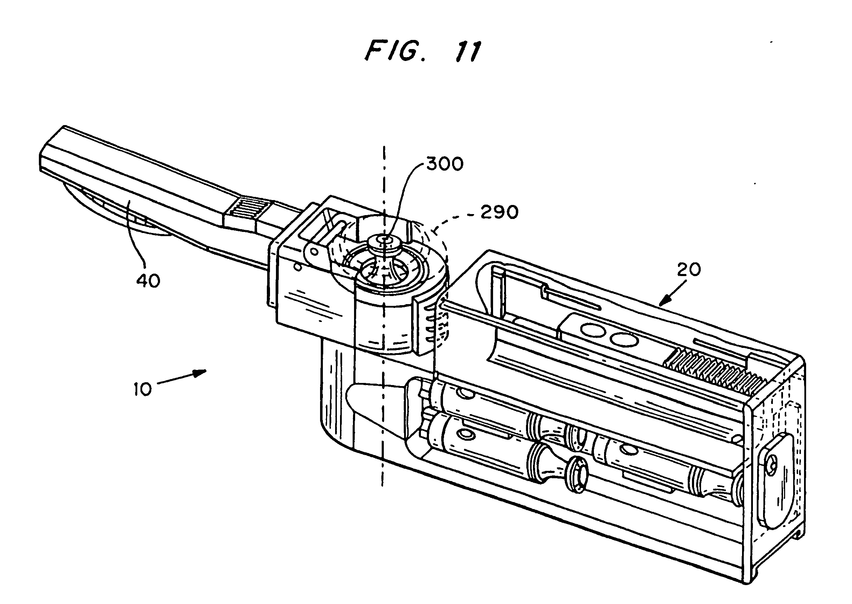

Figure 9, Figure 10, and Figure 11 show one specific embodiment of the dry particle inhaler (10). In Figure 9, the cover (90) of the mouthpiece is closed, and several capsule (300) are in the storage section (470). In Figure 10, the mouthpiece (40) has been rotated relative to the intake section (20). The longitudinal axis (60) [not shown] of the intake section here makes an approximately ninety degree angle with the longitudinal axis (70) of the mouthpiece section. This permits the cover (290) for the mixing section to be opened. A medicament capsule (300) taken from the storage section (470) is about to be inserted into the mixing section (30). In Figure 11, the mouthpiece (40) has been rotated to a fully extended position, the cover (290) for the mixing section has been closed, and the dry particle inhaler 910) is ready for use. In one embodiment of the dry particle inhaler (10), when the dry particle inhaler is in the closed position (Figure 9), the interior of the intake section (20) would be isolated from the outside air, but the mouthpiece (40) interior and the mixing section interior (291) would not be, permitting them to dry out after being exposed to the humid breath of a user.

Figure 12, Figure 13, Figure 14, and Figure 15 show a temporal sequence where a capsule (300) of medicament is loaded into the mixing section (30) of a dry particle inhaler (10), and the mouthpiece (40) is extended for use. The dry particle inhaler (10) described herein can also be used for nasal delivery of medicaments. A small tube (not shown) can be fitted to the end of the mouthpiece (40), and the other end of the tube inserted into the nostril. Alternatively, the mouthpiece (40) may be replaced by a nosepiece (not shown), whose free end is sized to be inserted into a nostril of a user. In another embodiment, a device such as a bellows or a syringe is used to force air through the dry particle inhaler (10) into a nosepiece inserted into the nostril of a user (not shown).

Figure 16 shows the fluid (air) flow of the dry particle inhaler (10) modeled as the equivalent electrical circuit. This is styled a "pneumatic resistance circuit".

Figure 17 shows a schematic view of the dry particle inhaler (10). The air passage (50) through the dry particle inhaler widens as it goes through the mouthpiece (40) along the direction of the air flow (460). The opening (135) of the mouthpiece to be inserted into the mouth of the user may be roughly ellipsoid, or oval, and thus have a major axis and a minor axis. The ratio of these two may be called the horizontal aspect ratio. In one embodiment of the invention, the horizontal aspect ratio is between 2:1 and 4:1. In one embodiment of the dry particle inhaler (10), the horizontal aspect ratio is 3:1. Shaping the opening (135) in this manner keeps the drug particles collimated, maintains the optimal velocity of the particles in the air stream, and is oriented to the natural horizontal aspect ratio of the oropharyngeal region of the mouth. In one embodiment of the invention, the outline of the opening (135) resembles a bean.

The dry particle inhaler described herein may be used with medicament particles of low, medium, and high shear forces.

The dry particle inhaler and capsules described herein may be made with a variety of suitable materials known to those skilled in the art, such as metal, glass, rubber, and plastic.

## Claims

1. A dry powder inhaler (10) comprising:
(a) an intake section (20) in fluid communication with
(b) a mixing section (30) in fluid communication with
(c) a mouthpiece (40) or nose-piece including an opening (135);
wherein the intake section (20) and the mouthpiece (40) or nose-piece each have a longitudinal axis (60, 70); and
wherein in use of the inhaler air flows through a passage (50) extending from the intake section (20) through the mixing section (30) through the mouthpiece (40) or nose-piece, **characterised in that**:
(i) the mixing section includes a Venturi for regulating the flow rate of air in the mixing section;
(ii) the mixing section includes one or more cyclone-creating structures; and
(iii) the intake section includes a piston and spring (220) combination, for modulating the flow of air through the inhaler (10),
such that in use of the inhaler (10) a user generates a vacuum in his/her lungs before air flows through the inhaler, at which time an air flow occurs in the inhaler that draws a medicament dose out of the inhaler via the mouthpiece (40) or nose-piece.

2. The dry powder inhaler of Claim 1 wherein the mouthpiece (4) and the mixing section (30) are mechanically connected by a swivel joint (80) which allows the longitudinal axis (60) of the intake section to be parallel to the longitudinal axis (70) of the mouthpiece.

3. The dry powder inhaler of Claim 1 further comprising a cover (90; 290) mechanically connected to the dry powder inhaler (10), wherein the cover (90; 290) shelters the mixing section (30) or mouthpiece (40).

4. The dry powder inhaler of Claim 3 including a swivel joint wherein the mixing section cover (290) only opens when the angle defined by the longitudinal axis (60) of the intake section and the longitudinal axis (70) of the mouthpiece and the swivel joint vertex is a fixed number of degrees.

5. The dry powder inhaler of Claim 4; wherein the fixed number of degrees the angle must subtend to permit opening of the Cover (290) is between approximately ninety degrees and one hundred and eighty degrees.

6. The dry powder inhaler of Claim 1 further comprising a storage section (470) mechanically connected to the dry powder inhaler, wherein a cover (480) mechanically connected to the storage section (470) shelters the storage section, and the cover may assume one or more fixed open positions.

7. The dry powder inhaler of Claim 3 or Claim 6 wherein the mixing or storage section cover (290; 480) is translucent and is a magnifying lens.

8. The dry powder inhaler of Claim 6 wherein the storage section (470) further includes mechanical fasteners mechanically connected to the storage section to secure capsules within the storage section.

9. The dry powder inhaler of Claim 1 comprising a mouthpiece (40) sized to extend mid-way into the oral cavity of a user, the mouthpiece including a tongue depressor (120).

10. The dry powder inhaler of Claim 1 wherein the intake section (20) includes an inner channel and comprises:
(a) an intake port (140); coverable by
(b) the piston of the piston and spring combination; and
(c) a bleed orifice (160)
wherein the intake port (140) and the bleed orifice (160) both admit air to the dry powder inhaler (10), and regulate the rate of admission of the air.

11. The dry powder inhaler of Claim 1 wherein the mouthpiece opening (135) has an approximately 3:1 horizontal aspect ratio.

12. A dry powder inhaler according to Claim 1 wherein the piston and spring combination for modulating the flow of air through the inhaler comprises:
(α) a piston (170) comprising a piston head (180) connected to a piston rod (190); and
(β) one or more springs (220) connected to the piston (170) and the inner walls (230) of an intake chamber in the intake section;
wherein the piston head (180) generally covers the intake port (140); wherein the piston head (180) is movable towards and away from the intake port (140) to regulate the admission of air via the intake port (140), and wherein movement of the piston head (180) is modulated by the springs (220).

13. A dry powder inhaler according to Claim 12 wherein the piston rod (190) is wider at a proximal portion connected to the piston head (180) and narrower at a distal portion remote therefrom; and wherein the piston rod extends through an orifice in the intake chamber such that movement of the piston rod further regulates the rate of admission of air.

14. A dry powder inhaler according to Claim 12 comprising a feedback module (240) connected to the intake chamber, wherein the feedback module (240) generates an electrical signal in response to the flow of air in the intake chamber.

15. The dry powder inhaler of Claim 14, wherein the feedback module (240) generates an audio signal.

16. The dry powder inhaler of Claim 14 wherein the strength of the signal from the feedback module (240) may be varied by a user of the dry powder inhaler.

17. A dry powder inhaler according to Claim 2, wherein the mixing section (30) is a chamber which comprises a holder (260) for a capsule (300) having at least one of top and bottom keying portions (360, 430); and a holder (260) is nested inside the chamber; wherein the holder (260) is capable of mechanically gripping the top and bottom keying portions (360,430) of the capsule, and wherein the holder is capable of opening the capsule (300) when the angle defined by the longitudinal axis (60) of the intake section and the longitudinal axis (70) of the mouthpiece and the swivel joint vertex is a fixed number of degrees, and closing the capsule (300) when the angle defined by the longitudinal axis (60) of the intake section and the longitudinal axis (70) of the mouthpiece and the swivel joint vertex is a fixed number of degrees.

18. The dry powder inhaler of Claim 17, wherein the fixed number of degrees needed to open the capsule (300) is between approximately ninety degrees and one hundred and eighty degrees, and the fixed number of degrees to close the capsule (300) is between approximately ninety and zero degrees.

19. The dry powder inhaler of Claim 17, wherein the holder (260) only admits one capsule (300) with one type of keying portion.

20. A dry powder inhaler according to Claim 17 wherein the Venturi chamber comprises at least a tangential inlet or outlet that is shaped to give air passing through it a cyclonic flow.

21. The dry powder inhaler of Claim 1, wherein the Venturi chamber (270) is curved or includes protrusions or spiral shapes (280) to keep the air velocity below the inflection speed limit.

22. A dry powder inhaler according to Claim 1, including a capsule (300) for holding a medicament, comprising a keying surface (360,430) to align the capsule in the dry powder inhaler (10).

23. A dry powder inhaler according to Claim 1 including a capsule (300) for holding medicament, comprising an inlet and an outlet that are alignable to direct flow through the capsule in a cyclonic pattern.

24. A dry powder inhaler according to Claim 23 wherein the capsule comprises a first tube and a second tube, wherein:
(a) the first tube (310) has a long axis (340) and has an inner and an outer surface radially spaced from the long axis, wherein the tube is open at one end (320) perpendicular to the long axis and closed at one end (330) perpendicular to the long axis; and wherein the first tube has at least one protrusion (350) on its outer surface; and
(b) the second tube (380) has a long axis (410) and has an inner and an outer surface radially spaced from the long axis (410), wherein the tube (380) is open at one end (390) perpendicular to the long axis (410) and closed at one end (400) perpendicular to the long axis (410) and wherein the second tube (380) has at least one protrusion (420) on its inner surface; and wherein the outer circumference of the first tube (310) is approximately equal to the inner circumference of the second tube (380), such that the open end (320) of the first tube can slide snugly into the open end (390) of the second tube; and wherein the protrusion (350) on the outer surface of the first tube (310) may slide past the protrusion (420) or a slot on the inner surface of the second tube(380), locking the tubes together;
wherein the first tube (310)' and the second tube (380) each have one or more secondary holes (370,440) other than the openings (320,390) at the end of each tube, wherein at least one secondary hole (370) in the first tube (310) may be made coincident with at least one secondary hole 9440) in the second tube (380) when the first tube (310) is slid onto the second tube (380) in the unlocked position by rotation of the first and second tubes (310,380) about their long axes (340,440); and
wherein when the first tube (310) is locked onto the second tube (380) at least two secondary holes (370) in the first tube may be made coincident with at least two secondary holes (440) in the second tube by rotation of the first and second tubes (310,380) about their long axes (340,440).

25. A dry powder inhaler according to Claim 23 further comprising keying surfaces (360,430) at the closed ends of the tubes.

26. A dry powder inhaler according to Claim 23 comprising one or more structures (445) in the interior surface of the capsule, that creates cyclonic airflow.

27. A dry powder inhaler according to Claim 26, wherein the structures (445) are cone-shaped.

28. A dry powder inhaler according to Claim 24, the capsule of which contains medicament selected from the group consisting of liquid, powder, and gaseous medicaments.

29. A dry powder inhaler according to Claim 22, the capsule of which includes a fastening mechanism to attach it to a second medicament capsule.

30. A dry powder inhaler according to Claim 23 the capsule of which is attachable, by means of the fastening mechanism, to a storage compartment for capsules in an inhaler.

31. The dry powder inhaler of Claim 1, wherein the mixing section (30) has a long axis, and wherein the air flowing through the mixing section (30) to the mouthpiece (40) exits the mixing section (30) at a tangent to a circle described by a radius about the long axis of the mixing section (30).

## Patentansprüche

1. Trockenpulver-Inhalator (10) mit:
(a) einem Einlassabschnitt (20) in Fluidverbindung mit
(b) einem Mischabschnitt (30) in Fluidverbindung mit
(c) einem Mundstück (40) oder Nasenstück mit einer Öffnung (135),
wobei der Einlassabschnitt (20) und das Mundstück (40) oder Nasenstück jeweils eine Longitudinalachse (60,70) aufweisen, und
wobei beim Einsatz des Inhalators Luft durch einen Durchgang (50) strömt, der sich von dem Einlassabschnitt (20) über den Mischabschnitt (30) durch das Mundstück (40) oder das Nasenstück erstreckt, **dadurch gekennzeichnet, dass**:
(i) der Mischabschnitt ein(e) Venturi(-Kammer) zum Regeln der Strömungsrate von Luft in den Mischabschnitt aufweist,
(ii) der Mischabschnitt eine oder mehrere, einen Wirbelstrom erzeugende Strukturen aufweist,
(iii) der Einlassabschnitt eine Kolben- und Federkombination (220) zum Modulieren der Luftströmung durch den Inhalator (10) aufweist,
so dass beim Einsatz des Inhalators (10) ein Benutzer Unterdruck in seiner/ihrer Lunge erzeugt, bevor Luft durch den Inhalator strömt, wobei zu dieser Zeit eine Luftströmung in dem Inhalator auftritt, welche eine Medikamentendosis aus dem Inhalator über das Mundstück (40) oder das Nasenstück saugt.

2. Trockenpulver-Inhalator nach Anspruch 1, wobei das Mundstück (4) und der Mischabschnitt (30) durch ein Drehgelenk (80) mechanisch verbunden sind, das es gestattet, dass die Longitudinalachse (60) des Einlassabschnitts parallel zu der Longitudinalachse (70) des Mundstücks ist.

3. Trockenpulver-Inhalator nach Anspruch 1, ferner mit einer Abdeckung (90;290), die mit dem Trockenpulver-Inhalator (10) mechanisch verbunden ist, wobei die Abdeckung (90;290) den Mischabschnitt (30) oder das Mundstück (40) bedeckt.

4. Trockenpulver-Inhalator nach Anspruch 3, mit einem Drehgelenk, wobei die Abdeckung (290) des Mischabschnitts sich nur öffnet, wenn der von der Longitudinalachse (60) des Einlassabschnitts und der Longitudinalachse (70) des Mundstücks und dem Scheitel des Drehgelenks festgelegte Winkel eine feststehende Gradzahl hat.

5. Trockenpulver-Inhalator nach Anspruch 4, wobei die feststehende Gradzahl, über die sich der Winkel erstrecken muss, um ein Öffnen der Abdeckung (290) zu ermöglichen, zwischen etwa neunzig Grad und hundertachtzig Grad liegt.

6. Trockenpulver-Inhalator nach Anspruch 1, ferner mit einem Speicherabschnitt (470), der mit dem Trockenpulver-Inhalator mechanisch gekoppelt ist, wobei eine Abdeckung (480), die mit dem Speicherabschnitt (470) mechanisch verbunden ist, den Speicherabschnitt bedeckt, und wobei die Abdeckung eine oder mehrere feststehende offene Positionen annehmen kann.

7. Trockenpulver-Inhalator nach Anspruch 3 oder Anspruch 6, wobei die Mischungs- oder Speicherabschnitt-Abdeckung (290;480) lichtdurchlässig ist und eine Vergrößerungslinse ist.

8. Trockenpulver-Inhalator nach Anspruch 6, wobei der Speicherabschnitt (470) ferner mechanische Befestiger aufweist, die mit dem Speicherabschnitt mechanisch verbunden sind, um Kapseln in dem Speicherabschnitt festzuhalten.

9. Trockenpulver-Inhalator nach Anspruch 1, mit einem Mundstück (40), das so bemessen ist, dass es sich halb in die Mundhöhle eines Benutzers erstreckt, wobei das Mundstück einen Zungendepressor (120) aufweist.

10. Trockenpulver-Inhalator nach Anspruch 1, wobei der Einlassabschnitt (20) einen inneren Kanal aufweist und umfasst:
(a) eine Einlassöffnung (140), die bedeckbar ist durch
(b) den Kolben der Kolben- und Federkombination, und
(c) eine Ablassöffnung (160),
wobei die Einlassöffnung (140) und Ablassöffnung (160) beide Luft in den Trockenpulver-Inhalator (10) lassen und die Luftzutrittsrate regeln.

11. Trockenpulver-Inhalator nach Anspruch 1, wobei die Mundstücköffnung (135) ein horizontales Seitenverhältnis von etwa 3 : 1 aufweist.

12. Trockenpulver-Inhalator nach Anspruch 1, wobei die Kolben- und Federkombination zum Modulieren der Luftströmung durch den Inhalator umfasst:
(α) einen Kolben (170), der einen mit einer Kolbenstange (190) verbundenen Kolbenkopf (180) umfasst, und
(β) eine oder mehrere Federn (220), die mit dem Kolben (170) und den Innenwänden (230) einer Einlasskammer in dem Einlassabschnitt verbunden sind,
wobei der Kolbenkopf (180) die Einlassöffnung(140) bedeckt, wobei der Kolbenkopf (180) zu der Einlassöffnung (140) hin und von dieser weg bewegbar ist, um den Zutritt von Luft über die Einlassöffnung (140) zu regeln, und wobei eine Bewegung des Kolbenkopfs (180) durch die Federn (220) moduliert ist.

13. Trockenpulver-Inhalator nach Anspruch 12, wobei die Kolbenstange (190) an einem mit dem Kolbenkopf (180) verbundenen proximalen Abschnitt breiter ist und an einem von diesem entfernten distalen Abschnitt schmäler ist, und wobei sich die Kolbenstange durch eine Öffnung in der Einlasskammer derart erstreckt, dass eine Bewegung der Kolbenstange die Luftzutrittsrate weiter regelt.

14. Trockenpulver-Inhalator nach Anspruch 12, mit einem Rückkoppelungsmodul (240), das mit der Einlasskammer verbunden ist, wobei das Rückkoppelungsmodul (240) ein elektrisches Signal in Reaktion auf die Luftströmung in der Einlasskammer erzeugt.

15. Trockenpulver-Inhalator nach Anspruch 14, wobei das Rückkoppelungsmodul (240) ein Audiosignal erzeugt.

16. Trockenpulver-Inhalator nach Anspruch 14, wobei die Stärke des Signals von dem Rückkoppelungsmodul (240) durch einen Benutzer des Trockenpulver-Inhalators variiert werden kann.

17. Trockenpulver-Inhalator nach Anspruch 2, wobei der Mischabschnitt (30) eine Kammer ist, die einen Halter (260) für eine Kapsel (300) mit mindestens einem oberen und unteren Verkeilungsabschnitt(en) (360,430)) aufweist, und ein Halter (260) in der Kammer untergebracht ist, wobei der Halter (260) die oberen und unteren Verkeilungsabschnitte (360,430) der Kapsel mechanisch greifen kann, und wobei der Halter die Kapsel (300) öffnen kann, wenn der zwischen der Longitudinalachse (60) des Einlassabschnitts (20) und der Longitudinalachse (70) des Mundstücks und des Drehgelenkscheitels eine feststehende Gradzahl aufweist und die Kapsel (300) schließt, wenn der zwischen der Longitudinalachse (60) des Einlassabschnitts und der Longitudinalachse (70) des Mundstücks sowie dem Drehgelenkscheitel festgelegte Winkel eine feststehende Gradzahl aufweist.

18. Trockenpulver-Inhalator nach Anspruch 17, wobei die feststehende Gradzahl, die zum Öffnen der Kapsel (300) benötigt wird, zwischen etwa neunzig Grad und hundertachtzig Grad liegt, und die feststehende Gradzahl, um die Kapsel (300) zu schließen, zwischen etwa neunzig und null liegt.

19. Trockenpulver-Inhalator nach Anspruch 17, wobei der Halter (260) nur eine Kapsel (300) mit einer Art Verkeilungsabschnitt zulässt.

20. Trockenpulver-Inhalator nach Anspruch 17, wobei die Venturi-Kammer mindestens einen Tangentialeinlass oder -auslass umfasst, der so geformt ist, dass der durch sie strömenden Luft eine Wirbelströmung vermittelt wird.

21. Trockenpulver-Inhalator nach Anspruch 1, wobei die Venturi-Kammer (270) gekrümmt ist oder Vorsprünge oder Spiralformen (280) aufweist, um die Luftgeschwindigkeit unter dem Flexionsgeschwindigkeitslimit zu halten.

22. Trockenpulver-Inhalator nach Anspruch 1, mit einer Kapsel (300) zum Halten eines Medikaments, die eine Verkeilungsfläche (360,430) aufweist, um die Kapsel in dem Trockenpulver-Inhalator (10) auszurichten.

23. Trockenpulver-Inhalator nach Anspruch 1, mit einer Kapsel (300) zum Halten eines Medikaments, umfassend einen Einlass und einen Auslass, die ausrichtbar sind, um eine Strömung durch die Kapsel in einem Wirbelstrommuster zu leiten.

24. Trockenpulver-Inhalator nach Anspruch 23, wobei die Kapsel ein erstes Rohr und ein zweites Rohr umfasst, wobei:
(a) das erste Rohr (310) eine Langachse (340) aufweist und eine Innen- und Außenfläche radial von der Langachse beabstandet hat, wobei das Rohr an einem Ende (320) senkrecht zu der Langachse offen ist und an einem Ende (330) senkrecht zu der Langachse geschlossen ist, und wobei das erste Rohr mindestens einen Vorsprung (350) an seiner Außenfläche aufweist, und
(b) das zweite Rohr (380) eine Langachse (410) aufweist und eine Innen- und Außenfläche radial von der Langachse (410) beabstandet hat, wobei das Rohr (380) an einem Ende (390) senkrecht zu der Langachse (410) offen ist und an einem Ende (400) senkrecht zu der Langachse (410) geschlossen ist, und wobei das zweite Rohr (380) mindestens einen Vorsprung (420) an seiner Innenfläche aufweist, und wobei der Außenumfang des ersten Rohrs (310) in etwa gleich dem Innenumfang des zweiten Rohrs (380) ist, so dass das offene Ende (320) des ersten Rohrs leicht in das offene Ende (390) des zweiten Rohrs gleiten kann, und wobei der Vorsprung (350) an der Außenfläche des ersten Rohrs (310) an dem Vorsprung (420) oder an einem Schlitz an der Innenfläche des zweiten Rohrs (380) vorbeigleiten kann, wodurch die Rohre miteinander verriegelt werden,
wobei das erste Rohr (310) und das zweite Rohr (380) jeweils eine oder mehrere Sekundärlöcher (370,440) außer den Öffnungen (320,390) am Ende jedes Rohrs aufweisen, wobei mindestens ein Sekundärloch (370) in/an dem ersten Rohr (310) zum Koinzidieren mit mindestens einem Sekundärloch (440) in/an dem zweiten Rohr (380) gebracht werden kann, wenn das erste Rohr (310) auf das zweite Rohr (380) in der unverriegelten Position durch Drehung der ersten und zweiten Rohre (310,380) um ihre Langachsen (340,440) geschoben wird, und
wobei, wenn das erste Rohr (310) am zweiten Rohr (380) verriegelt wird, mindestens zwei Sekundärlöcher (370) in dem ersten Rohr mit mindestens zwei Sekundärlöchern (440) in dem zweiten Rohr durch Drehen der ersten und zweiten Rohre (310,380) um ihre Langachsen (340,440) zum Koinzidieren gebracht werden kann.

25. Trockenpulver-Inhalator nach Anspruch 23, ferner mit Verkeilungsflächen (360,430) an den geschlossenen Enden der Rohre.

26. Trockenpulver-Inhalator nach Anspruch 23, mit einer oder mehreren Strukturen (445) an der Innenfläche der Kapsel, die eine Wirbelluftströmung erzeugt.

27. Trockenpulver-Inhalator nach Anspruch 26, wobei die Strukturen (445) konusförmig sind.

28. Trockenpulver-Inhalator nach Anspruch 24, wobei dessen Kapsel ein Medikament enthält, das aus der aus flüssigen, pulverförmigen und gasförmigen Medikamenten bestehenden Gruppe ausgewählt ist.

29. Trockenpulver-Inhalator nach Anspruch 22, wobei dessen Kapsel einen Befestigungsmechanismus aufweist, um sie an einer zweiten Medikamentenkapsel anzubringen.

30. Trockenpulver-Inhalator nach Anspruch 23, wobei dessen Kapsel mittels des Befestigungsmechanismus an einem Lagerfach für Kapseln in einem Inhalator anbringbar ist.

31. Trockenpulver-Inhalator nach Anspruch 1, wobei der Mischabschnitt (30) eine Langachse aufweist und wobei die durch den Mischabschnitt (30) zu den Mundstücken (40) strömende Luft den Mischabschnitt (30) in einer Tangente zu einem Kreis verlässt, der von einem Radius um die Langachse des Mischabschnitts (30) beschrieben wird.

## Revendications

1. Inhalateur à poudre sèche (10) comprenant :
(a) une partie d'admission (20) en communication fluide avec
(b) une partie de mélange (30) en communication fluide avec
(c) un embout buccal (40) ou un embout nasal comprenant une ouverture (135) ;
dans lequel la partie d'admission (20) et l'embout buccal (40) ou l'embout nasal ont chacun un axe longitudinal (60, 70) ; et
dans lequel, lors de l'utilisation de l'inhalateur, de l'air s'écoule par un passage (50) s'étendant depuis la partie d'admission (20) à travers la partie de mélange (30) à travers l'embout buccal (40) ou l'embout nasal, **caractérisé en ce que** :
(i) la partie de mélange comprend un diffuseur destiné à réguler le débit d'air dans la partie de mélange,
(ii) la partie de mélange comprend une ou plusieurs structures de formation de cyclone ; et
(iii) la partie d'admission comprend une combinaison de piston et de ressort (220), destinée à moduler l'écoulement d'air à travers l'inhalateur (10),
de sorte que, lors de l'utilisation de l'inhalateur (10), un utilisateur génère un vide dans ses poumons avant que l'air ne s'écoule à travers l'inhalateur, moment auquel un écoulement d'air se produit dans l'inhalateur qui aspire une dose de médicament provenant de l'inhalateur par le biais de l'embout buccal (40) ou de l'embout nasal.

2. Inhalateur à poudre sèche selon la revendication 1, dans lequel l'embout buccal (40) et la partie de mélange (30) sont raccordés mécaniquement par un joint articulé (80) qui permet à l'axe longitudinal (60) de la partie d'admission d'être parallèle à l'axe longitudinal (70) de l'embout buccal.

3. Inhalateur à poudre sèche selon la revendication 1, comprenant en outre un couvercle (90 ; 290) raccordé mécaniquement à l'inhalateur à poudre sèche (10), dans lequel le couvercle (90 ; 290) protège la partie de mélange (30) ou l'embout buccal (40).

4. Inhalateur à poudre sèche selon la revendication 3, comprenant un joint articulé dans lequel le couvercle (290) de la partie de mélange s'ouvre seulement lorsque l'angle défini par l'axe longitudinal (60) de la partie d'admission et l'axe longitudinal (70) de l'embout buccal et du sommet du joint articulé correspond à un nombre de degrés fixe.

5. Inhalateur à poudre sèche selon la revendication 4, dans lequel le nombre de degrés fixe que l'angle doit avoir pour permettre l'ouverture du couvercle (290) se situe entre approximativement 90 degrés et 180 degrés.

6. Inhalateur à poudre sèche selon la revendication 1, comprenant en outre une partie de stockage (470) raccordée mécaniquement à l'inhalateur à poudre sèche, dans lequel un couvercle (480) raccordé mécaniquement à la partie de stockage (470) protège la partie de stockage, et le couvercle peut prendre une ou plusieurs positions ouvertes fixes.

7. Inhalateur à poudre sèche selon la revendication 3 ou la revendication 6, dans lequel le couvercle de la partie de stockage ou de mélange (290 ; 480) est translucide et est une lentille grossissante.

8. Inhalateur à poudre sèche de la revendication 6, dans lequel la partie de stockage (470) comprend en outre des dispositifs de fixation mécaniques raccordés mécaniquement à la partie de stockage afin de fixer des capsules à l'intérieur de la partie de stockage.

9. Inhalateur à poudre sèche selon la revendication 1, comprenant un embout buccal (40) calibré pour s'étendre à mi-chemin dans la cavité orale d'un utilisateur, l'embout buccal comprenant un abaisse-langue (120).

10. Inhalateur à poudre sèche selon la revendication 1, dans lequel la partie d'admission (20) comprend un canal interne et comprend :
(a) un orifice d'admission (140) ; pouvant être couvert par
(b) le piston de la combinaison de piston et de ressort ; et
(c) un orifice de prélèvement (160)
dans lequel l'orifice d'admission (140) et l'orifice de prélèvement (160) admettent tous deux de l'air vers l'inhalateur à poudre sèche (10), et régulent le débit d'admission de l'air.

11. Inhalateur à poudre sèche selon la revendication 1, dans lequel l'ouverture (135) de l'embout buccal a un rapport d'aspect horizontal d'approximativement 3:1.

12. Inhalateur à poudre sèche selon la revendication 1, dans lequel la combinaison de piston et de ressort destinée à moduler l'écoulement d'air à travers l'inhalateur comprend :
(α) un piston (170) comprenant une tête de piston (180) raccordée à une tige de piston (190) ; et
(β) un ou plusieurs ressorts (220) raccordés au piston (170) et aux parois internes (230) d'une chambre d'admission dans la partie d'admission ;
dans lequel la tête de piston (180) recouvre généralement l'orifice d'admission (140) ; dans lequel, la tête de piston (180) peut être déplacée vers et écartée de l'orifice d'admission (140) afin de réguler l'admission d'air par le biais de l'orifice d'admission (140), et dans lequel un mouvement de la tête de piston (180) est modulé par les ressorts (220).

13. Inhalateur à poudre sèche selon la revendication 12, dans lequel la tige de piston (190) est plus large au niveau d'une partie proximale raccordée à la tête de piston (180) et plus étroite au niveau d'une partie distale éloignée de celle-ci ; et dans lequel la tige de piston s'étend à travers un orifice dans la chambre d'admission de telle sorte que le mouvement de la tige de piston régule davantage le débit d'admission d'air.

14. Inhalateur à poudre sèche selon la revendication 12, comprenant un module de retour (240) raccordé à la chambre d'admission, dans lequel le module de retour (240) génère un signal électrique en réponse à l'écoulement d'air dans la chambre d'admission.

15. Inhalateur à poudre sèche selon la revendication 14, dans lequel le module de retour (240) génère un signal audio.

16. Inhalateur à poudre sèche selon la revendication 14, dans lequel la force du signal provenant du module de retour (240) peut être modifiée par.un utilisateur de l'inhalateur à poudre sèche.

17. Inhalateur à poudre sèche selon la revendication 2, dans lequel la partie de mélange (30) est une chambre qui comprend un support (260) pour une capsule (300) ayant au moins une parmi des parties de manipulation de sommet et de fond (360, 430) ; et un support (260) est logé à l'intérieur de la chambre, dans lequel le support (260) est capable de saisir mécaniquement les parties de manipulation de sommet et de fond (360, 430) de la capsule, et dans lequel le support est capable d'ouvrir la capsule (300) lorsque l'angle défini par l'axe longitudinal (60) de la partie d'admission et l'axe longitudinal (70) de l'embout buccal et du sommet du joint articulé est un nombre de degrés fixe, et de fermer la capsule (300) lorsque l'angle défini par l'axe longitudinal (60) de la partie d'admission et l'axe longitudinal (70) de l'embout buccal et du sommet du joint articulé est un nombre de degrés fixe.

18. Inhalateur à poudre sèche selon la revendication 17, dans lequel le nombre de degrés fixe nécessaire pour ouvrir la capsule (300) se situe entre approximativement 90 degrés et 180 degrés, et le nombre de degrés fixe pour fermer la capsule (300) se situe entre approximativement 90 degrés et 0 degré.

19. Inhalateur à poudre sèche selon la revendication 17, dans lequel le support (260) admet seulement une capsule (300) avec un type de partie de manipulation.

20. Inhalateur à poudre sèche selon la revendication 17, dans lequel la chambre de diffuseur comprend au moins une entrée ou une sortie tangentielle qui est formée pour donner à l'air passant à travers celle-ci un écoulement cyclonique.

21. Inhalateur à poudre sèche selon la revendication 1, dans lequel la chambre du diffuseur (270) est courbée ou comprend des saillies ou des formes en spirale (280) afin de maintenir la vitesse de l'air en dessous de la limite de vitesse d'inflexion.

22. Inhalateur à poudre sèche selon la revendication 1, comprenant une capsule (300) destinée à contenir un médicament, comprenant une surface de manipulation (360, 430) pour aligner la capsule dans l'inhalateur à poudre sèche (10).

23. Inhalateur à poudre sèche selon la revendication 1, comprenant une capsule (300) destinée à contenir un médicament, comprenant une entrée et une sortie qui peuvent être alignées de manière à diriger l'écoulement à travers la capsule dans un schéma cyclonique.

24. Inhalateur à poudre sèche selon la revendication 23, dans lequel la capsule comprend un premier tube et un second tube, dans lequel :
(a) le premier tube (310) a un long axe (340) et a des surfaces intérieure et extérieure espacées du long axe de manière radiale, dans lequel le tube est ouvert à une extrémité (320) perpendiculaire au long axe et fermé à une extrémité (330) perpendiculaire au long axe ; et dans lequel le premier tube a au moins une saillie (350) sur sa surface extérieure ; et
(b) le second tube (380) a un long axe (410) et a des surfaces intérieure et extérieure espacées du long axe (410) de manière radiale, dans lequel le tube (380) est ouvert à une extrémité (390) perpendiculaire au long axe (410) et fermé à une extrémité (400) perpendiculaire au long axe (410) et dans lequel le second tube (380) a au moins une saillie (420) sur sa surface intérieure ; et dans lequel la circonférence extérieure du premier tube (310) est approximativement égale à la circonférence intérieure du second tube (380), de telle sorte que l'extrémité ouverte (320) du premier tube peut coulisser de manière serrée dans l'extrémité ouverte (390) du second tube ; et dans lequel la saillie (350) sur la surface extérieure du premier tube (310) peut coulisser devant la saillie (420) ou une fente sur la surface intérieure du second tube (380), ce qui verrouille les tubes l'un avec l'autre ;
dans lequel le premier tube (310) et le second tube (380) comportent chacun un ou plusieurs trous secondaires (370, 440) autres que les ouvertures (320, 390) au niveau de l'extrémité de chaque tube, dans lequel au moins un trou secondaire (370) dans le premier tube (310) peut être rendu coïncidant avec au moins un trou secondaire (440) dans le second tube (380) lorsque le premier tube (310) coulisse sur le second tube (380) dans la position non verrouillée par rotation des premier et second tubes (310, 380) autour de leur long axe (340, 440) ; et
dans lequel, lorsque le premier tube (310) est verrouillé sur le second tube (380), au moins deux trous secondaires (370) dans le premier tube peuvent être rendus coïncidant avec au moins deux trous secondaires (440) dans le second tube par rotation des premier et second tubes (310, 380) autour de leur long axe (340, 440).

25. Inhalateur à poudre sèche selon la revendication 23, comprenant en outre des surfaces de manipulation (360, 430) au niveau des extrémités fermées des tubes.

26. Inhalateur à poudre sèche selon la revendication 23, comprenant une ou plusieurs structures (445) dans la surface intérieure de la capsule qui crée(nt) un écoulement d'air cyclonique.

27. Inhalateur à poudre sèche selon la revendication 26, dans lequel les structures (445) sont en forme de cône.

28. Inhalateur à poudre sèche selon la revendication 24, dont la capsule contient un médicament choisi dans le groupe comprenant des médicaments liquides, en poudre et gazeux.

29. Inhalateur à poudre sèche selon la revendication 22, dont la capsule comprend mécanisme de fixation afin de la fixer à une seconde capsule de médicament.

30. Inhalateur à poudre sèche selon la revendication 23, dont la capsule peut être fixe, au moyen du mécanisme de fixation, à un compartiment de stockage pour des capsules dans un inhalateur.

31. Inhalateur à poudre sèche selon la revendication 1, dans lequel la partie de mélange (30) a un long axe, et dans lequel l'écoulement d'air à travers la partie de mélange (30) vers l'embout buccal (40) sort de la partie de mélange (30) à une tangente d'un cercle décrit par un rayon autour du long axe de la partie de mélange (30).
